# EUROPEAN PATENT APPLICATION

(11) **EP 4 219 537 A1**
(43) Date of publication of application: **02.08.2023**
(21) Application number: 21871452.5
(22) Date of filing: 18.09.2021
(51) Int. Cl.: C07K 14/505, C12N 15/16, A61K 38/18, A61K 38/00

(54) **GLYCOSYLATION-MODIFIED ERYTHOPOIETIN AND USE THEREOF**

(30) Priority: 22.09.2020 CN 202011005340
(71) Applicant: Jecho Laboratories, Inc., Frederick District, MD 21704 (US); Jecho Biopharmaceuticals Co., Ltd., Tianjin 300467 (CN); Jecho Institute Co., Ltd., Shanghai 201100 (CN)
(72) Inventor: ZHU, Jianwei, Tianjin 300467 (CN); JIANG, Hua, Tianjin 300467 (CN); XIE, Yueqing, Tianjin 300467 (CN); YANG, Kaiyong, Tianjin 300467 (CN); XIAO, Xiaodong, Tianjin 300467 (CN); WANG, Zhenyu, Tianjin 300467 (CN); WANG, Man, Tianjin 300467 (CN)
(74) Representative: Weickmann & Weickmann PartmbB
(86) International application number: PCT/CN2021/119332
(87) International publication number: WO 2022/063082

(57) **Abstract**

A glycosylation-modified erythropoietin, containing a glycan structure incorporated into an N-glycosylation site, wherein the glycan structure contains FA4G4L2S4.

## Description

### Technical Field

The disclosure herein relates to the field of biomedicine, and in particular to a glycosylation-modified erythropoietin and an application thereof.

### Background

Erythropoietin (EPO), a glycoprotein hormone, is one of the members of the hematopoietic cytokine superfamily, and functions to promote the proliferation and differentiation of erythrocytes and increase the hemoglobin concentration of the body. EPO exerts biological effects by binding to cell surface transmembrane receptors, namely, EPO receptors. When EPO binds to the EPO receptors, it can induce a change in receptor conformation, which brings two adjacent EPO receptors closer to each other to form a homodimer and activates JAK2 tyrosine kinase bound to a receptor cytoplasmic juxtamembrane region, resulting in phosphorylation of multiple tyrosine residues of the receptors. JAK2 can further phosphorylate STAT to initiate related gene expressions, thereby activating multiple downstream signaling pathways to bring about proliferation and differentiation of the erythrocyte system. Clinical trials have demonstrated that EPO shows a therapeutic effect on a variety of diseases, and is mainly used for anemia induced by various causes.

Some therapeutic erythropoietin medicaments have the defects of short half-life and high frequency of clinical administration, which seriously affect their use in patients. There is an extremely urgent need of developing an erythropoietin with a long half-life.

### Summary

In order to solve the current problem of short half-life of erythropoietin, the present application provides a glycosylation-modified erythropoietin and an application thereof, including the glycosylation-modified erythropoietin as well as a preparation method therefor and a pharmaceutical composition thereof; meanwhile, the present application provides an application of the glycosylation-modified erythropoietin in the preparation of a medicament for treating anemia and a method for prolonging the half-life of erythropoietin.

The present application provides a glycosylation-modified erythropoietin, comprising a glycan structure binding to an N-glycosylation site, the glycan structure comprising FA4G4L2S4, wherein F represents fucose, A represents N-acetylglucosamine, G represents galactose, L represents lactose, and S represents sialic acid.

In some embodiments, a ratio of the FA4G4L2S4 is 15% or more.

In some embodiments, the glycan structure comprises FA4G4L1S4, wherein F represents fucose, A represents N-acetylglucosamine, G represents galactose, L represents lactose, and S represents sialic acid.

In some embodiments, a ratio of the FA4G4L1S4 is 20% or more.

In some embodiments, the glycan structure comprises FA4G4S4, wherein F represents fucose, A represents N-acetylglucosamine, G represents galactose, and S represents sialic acid.

In some embodiments, a ratio of the FA4G4S4 is 10% or more.

In some embodiments, the glycan structure comprises Neu5Gc, a molar ratio of which is 0.5% or less.

In some embodiments, the glycosylation-modified erythropoietin comprises an amino acid sequence as set forth in SEQ ID NO: 1.

In some embodiments, the glycosylation-modified erythropoietin comprises N-glycosylation sites as follows: N24, N30, N38, N83, and N88.

In another aspect, the present application provides a preparation method for the glycosylation-modified erythropoietin, comprising the step of: under a condition of expressing the glycosylation-modified erythropoietin, culturing CHO-S cells comprising nucleic acid molecules encoding an amino acid sequence as set forth in SEQ ID NO: 1.

In another aspect, the present application provides a pharmaceutical composition, comprising the glycosylation-modified erythropoietin, and a pharmaceutically acceptable adjuvant.

In another aspect, the present application provides a use of the glycosylation-modified erythropoietin and/or the pharmaceutical composition in the preparation of a medicament for treating anemia.

In another aspect, the present application provides a use of the glycosylation-modified erythropoietin and/or the pharmaceutical composition in the preparation of a medicament for treating anemia, the anemia comprising renal anemia, multiple myeloma anemia and/or carcinogenic anemia.

In another aspect, the present application provides a method for treating anemia, comprising administering the glycosylation-modified erythropoietin and/or the pharmaceutical composition at a therapeutically effective amount to a subject in need thereof.

In another aspect, the present application provides a method for treating anemia, comprising administering the glycosylation-modified erythropoietin and/or the pharmaceutical composition at a therapeutically effective amount to a subject in need thereof, the anemia comprising renal anemia, multiple myeloma anemia and/or carcinogenic anemia.

In another aspect, the present application provides the glycosylation-modified erythropoietin and/or the pharmaceutical composition for use in treatment of anemia.

In another aspect, the present application provides the glycosylation-modified erythropoietin and/or the pharmaceutical composition for use in treatment of anemia, the anemia comprising renal anemia, multiple myeloma anemia and/or carcinogenic anemia.

In another aspect, the present application provides a method for prolonging the half-life of erythropoietin, comprising the step of: administering the glycosylation-modified erythropoietin to a subject in need thereof.

With increased half-life, the glycosylation-modified erythropoietin provided by the present application has the beneficial effect of increasing hemoglobin content, erythrocyte level, hematocrit value and/or reticulocyte level.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### Brief Description of Figures

The specific features of the present invention involved in the present application are listed in the appended claims. The characteristics and advantages of the present invention involved in the present application may be better understood by referring to the exemplary embodiments and the accompanying drawings described in detail below. The accompanying drawings are briefly illustrated as follows:

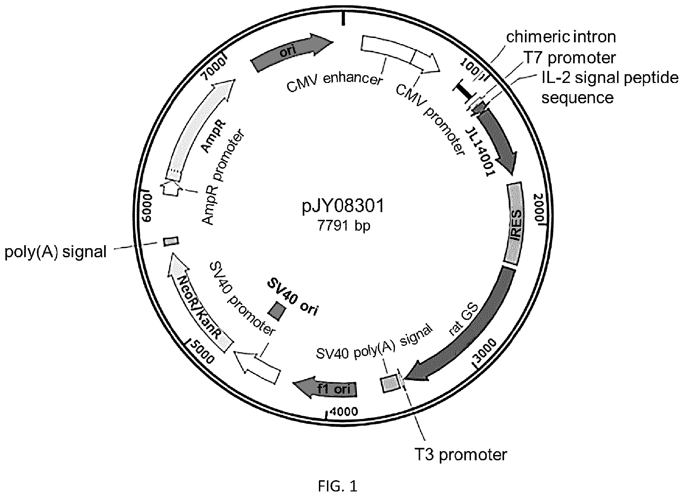

FIG. 1 shows a schematic structural diagram of an expression vector fragment of a glycosylation-modified erythropoietin according to the present application;

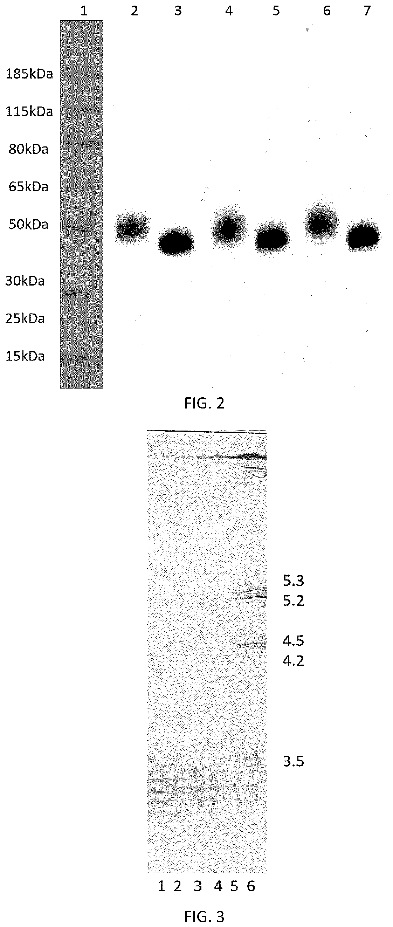

FIG. 2 shows the Western-Blot results of the glycosylation-modified erythropoietin according to the present application, with Lane 1 for a protein marker of a known molecular weight, Lanes 2, 4 and 6 respectively for three lots of purified products lot#20190302-2, lot#20190303-2 and lot#20190304-1 of the glycosylation-modified erythropoietin according to the present application, and Lanes 3, 5, and 7 for control groups Darbepoetin (Darbe, lot#1078765A);
FIG. 3 shows the IEF assay results of the glycosylation-modified erythropoietin according to the present application, with Lane 1 for a control group Darbepoetin (Darbe, lot#1078765A), Lanes 2, 3 and 4 respectively for three lots of purified products lot#20190302-2, lot#20190303-2 and lot#20190304-1 of the glycosylation-modified erythropoietin according to the present application, Lane 5 as blank, and Lane 6 for a protein marker with known pl;

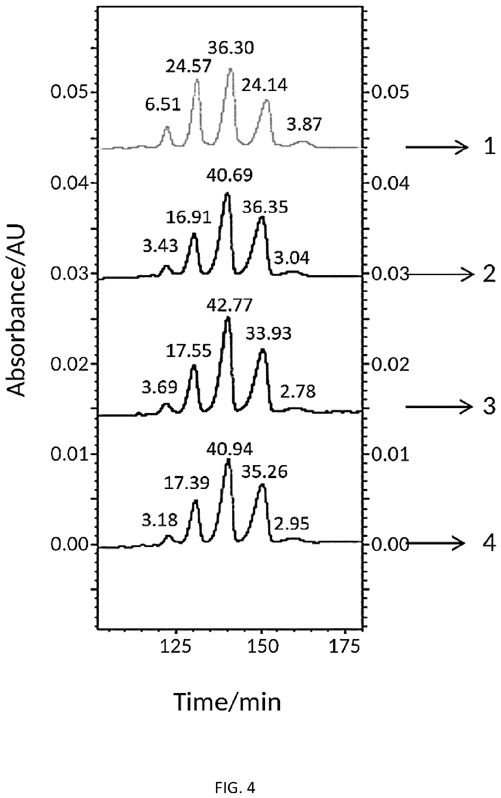

FIG. 4 shows the CZE assay results of the glycosylation-modified erythropoietin according to the present application, with Line 1 for a control group Darbepoetin (Darbe, lot#1078765A), and Lines 2, 3, and 4 respectively for three lots of purified products lot#20190302-2, lot#20190303-2 and lot#20190304-1 of the glycosylation-modified erythropoietin according to the present application;

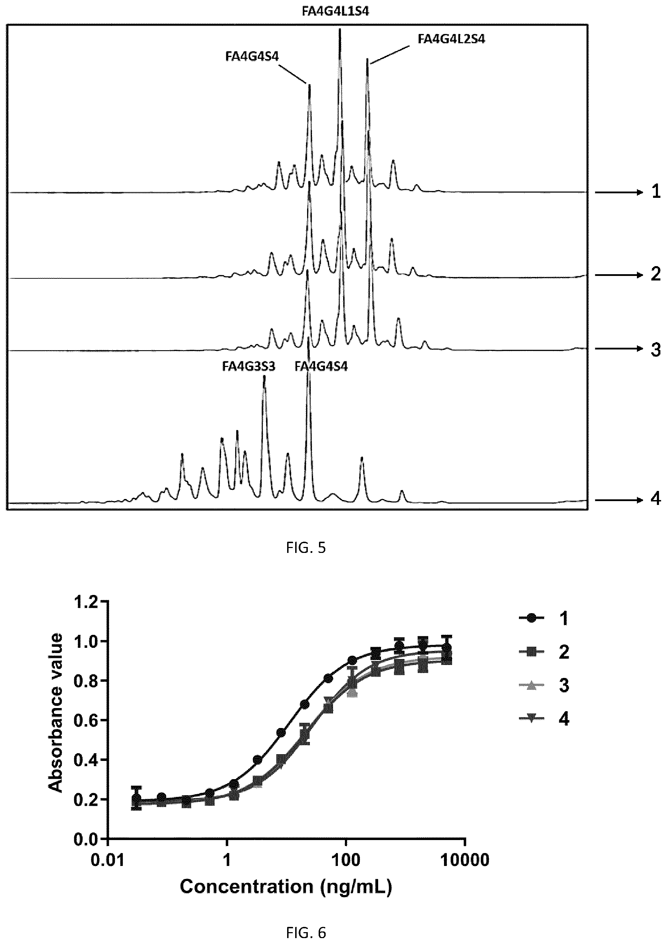

FIG. 5 shows the assay results of N-linked glycosylation composition of the glycosylation-modified erythropoietin according to the present application, with Lines 1, 2 and 3 respectively for three lots of purified products lot#20190302-2, lot#20190303-2 and lot#20190304-1 of the glycosylation-modified erythropoietin according to the present application, and Line 4 for a control group Darbepoetin (Darbe, lot#1078765A);
FIG. 6 shows the results of the effects of the glycosylation-modified erythropoietin according to the present application on the proliferative capacity of TF-1 cells, with 1 indicating a control group Darbepoetin (Darbe, lot#1078765A), and 2, 3 and 4 indicating three lots of purified products lot#20190302-2, lot#20190303-2 and lot#20190304-1 of the glycosylation-modified erythropoietin according to the present application, respectively;

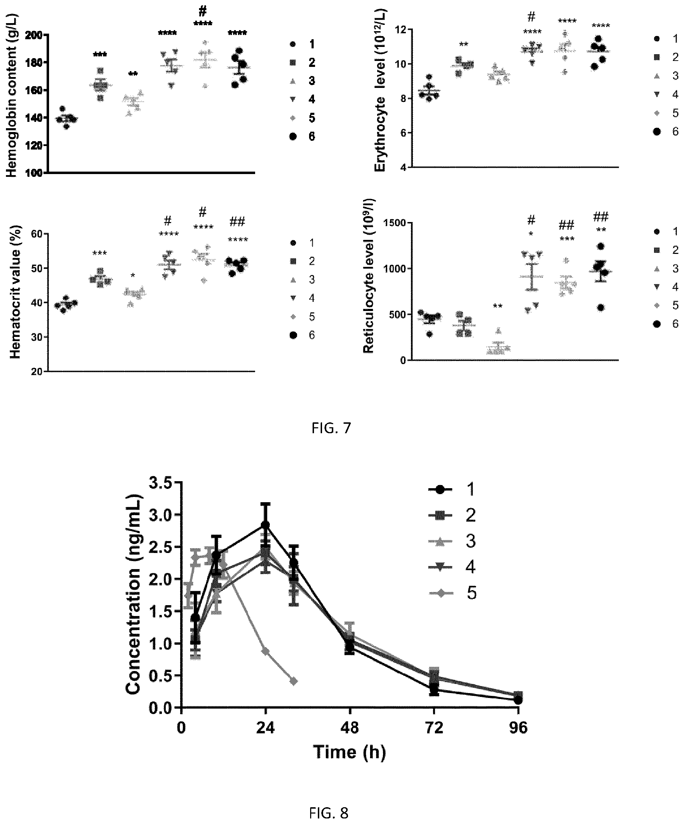

FIG. 7 shows the in vivo efficacy test results of the glycosylation-modified erythropoietin according to the present application, with 1 indicating a blank group (vehicle group), 2 indicating a control group A (Darbepoetin, Darbe, lot#1078765A), 3 indicating a control group B (EPO, Espo, lot#17Y03B), and 4, 5 and 6 respectively for three lots of purified products lot#20190302-2, lot#20190303-2 and lot#20190304-1 of the glycosylation-modified erythropoietin according to the present application, with * indicating the significance as compared with the blank group as follows: **** for P < 0.0001, *** for P < 0.001, ** for P < 0.01, and * for P < 0.05, and with # indicating the significance as compared with the control group B as follows: ## for P < 0.01, and # for P < 0.05; and
FIG. 8 shows the half-life assay results of the glycosylation-modified erythropoietin according to the present application, with 1 indicating a control group A (Darbepoetin, Darbe, lot#1078765A), 2, 3 and 4 respectively for three lots of purified products lot#20190302-2, lot#20190303-2 and lot#20190304-1 of the glycosylation-modified erythropoietin according to the present application, and 5 indicating a control group B (EPO, Espo, lot#17Y03B).

### Detailed Description

The embodiments of the invention of the present application will be illustrated by specific examples below. Those familiar with this technology can easily understand other advantages and effects of the invention of the present application from the content disclosed in the specification.

### TERMS & DEFINITIONS

In the present application, the term "half-life" or its abbreviation "T_{1/2}" is generally used to quantify the time taken for half of the dose of a medicament to be excreted by a subject.

In the present application, the term "glycan structure" generally refers to a polysaccharide or an oligosaccharide, i.e., a polymeric compound producing a plurality of monosaccharides after acid hydrolysis. A glycosylation-modified protein may comprise one or more glycan structures covalently coupled to the pendant group of a polypeptide chain via asparagine or arginine ("N-linked glycosylation") or via serine or threonine ("O-linked glycosylation"). For example, it is possible that a glycan structure of FA4G4L2S4 is attached to the N-glycosylation site of erythropoietin, a glycan structure of FA4G4L1S4 is attached to the N-glycosylation site of erythropoietin, a glycan structure of FA4G4S4 is attached to the N-glycosylation site of erythropoietin, or a glycan structure of Neu5Gc is attached to the N-glycosylation site of erythropoietin. The glycan structure is classified into two-antenna, three-antenna and four-antenna structures depending on their number of branchings (antennas). The glycan structure consists of a variety of monosaccharides, which, according to the Oxford nomenclature, include: fucose (abbreviated as Fuc or F), N-acetylglucosamine (abbreviated as GlcNAc, Gn or A), galactose (abbreviated as Gal or G), lactose (abbreviated as Lac or L), mannose (abbreviated as Man or M), N-acetylneuraminic acid (sialic acid, abbreviated as NANA, NeuSAc or S) and/or N-glycolylneuraminic acid (abbreviated as NGNA, Neu5Glc or Neu5Gc). For example, the term "FA4G4L2S4" refers to a glycan structure of a four-antenna structure including fucosacylation, 4 N-acetylglucosamines, 4 galactoses, 2 lactoses, and 4 sialic acids, with F representing the inclusion of a fucose modification, A representing N-acetylglucosamine, G representing galactose, L representing lactose, S representing sialic acid, the figure following A representing the number of the N-acetylglucosamine in one glycan structure, the figure following G representing the number of the galactose in one glycan structure, the figure following L representing the number of the lactose in one glycan structure, and the figure following S representing the number of the sialic acid in one glycan structure; the term "FA4G4L1S4" refers to a glycan structure of a four-antenna structure including fucosacylation, 4 N-acetylglucosamines, 4 galactoses, 1 lactose, and 4 sialic acids, with F representing the inclusion of a fucose modification, A representing N-acetylglucosamine, G representing galactose, L representing lactose, S representing sialic acid, the figure following A representing the number of the N-acetylglucosamine in one glycan structure, the figure following G representing the number of the galactose in one glycan structure, the figure following L representing the number of the lactose in one glycan structure, and the figure following S representing the number of the sialic acid in one glycan structure; the term "FA4G4S4" refers to a glycan structure of a four-antenna structure including fucosacylation, 4 N-acetylglucosamines, 4 galactoses, and 4 sialic acids, with F representing the inclusion of a fucose modification, A representing N-acetylglucosamine, G representing galactose, S representing sialic acid, the figure following A representing the number of the N-acetylglucosamine in one glycan structure, the figure following G representing the number of the galactose in one glycan structure, and the figure following S representing the number of the sialic acid in one glycan structure; and the term " Neu5Gc" refers to N-hydroxyacetylneuraminic acid.

In the present application, the term "N-glycosylation site" generally refers to a site, on a glycosylation-modified protein, containing asparagine or arginine for covalently linking the glycan structure. For example, the N-glycosylation site may be an asparagine residue used to covalently link the glycan structure to the glycosylation-modified protein. For example, the N-glycosylation site may be asparagine (Asn or N) residues at positions 24, 30, 38, 83 and 88 on the glycosylation-modified erythropoietin.

In the present application, the term "binding" of the glycan structure to the N-glycosylation site generally refers to a physical or chemical interaction between the glycan structure and the N-glycosylation site on the glycosylation-modified protein. For example, the binding may be direct or indirect linkage or attachment, the indirect linkage or attachment may be achieved by another biomolecule or compound, and the direct linkage or attachment may be covalent (for example, by chemical coupling) or non-covalent (for example, ionic interaction, hydrophobic interaction, hydrogen bond, etc.) binding or attachment. For example, the binding may be either covalent linkage between the glycan structure and the N-glycosylation site on the glycosylation-modified protein, or covalent linkage between a monosaccharide of the glycan structure and a free -NH2 group of an asparagine residue at the N-glycosylation site.

In the present application, the "ratio" of the glycan structure in the glycosylation-modified erythropoietin generally refers to the molar ratio of the glycan structure in the glycosylation-modified erythropoietin. The ratio can be analyzed by mass spectrometry after enzymolysis of the glycosylation-modified erythropoietin, and an exemplary mass spectrometry method may include combined high performance liquid chromatography-mass spectrometry (HPLC-MS). For example, in the glycosylation-modified erythropoietin, the ratio of the FA4G4L2S4 structure may be 15% or more, the ratio of the FA4G4L1S4 may be 20% or more, the ratio of the FA4G4S4 may be 10% or more, and the molar ratio of the Neu5Gc may be 0.5% or less.

In the present application, the term "molar ratio" is generally calculated and given as the molar ratio of a specific glycan structure = the molar number of the specific glycan structure/the molar number of all glycan structures in a protein, when all the glycan structures are released after glycosidase enzymolysis of glycoproteins. For example, the molar ratio may be the molar number of the structure of FA4G4L2S4/the molar number of all glycan structures in the glycosylation-modified erythropoietin, the molar number of FA4G4L1S4/the molar number of all glycan structures in the glycosylation-modified erythropoietin, the molar number of FA4G4S4/the molar number of all glycan structures in the glycosylation-modified erythropoietin, or the molar number of Neu5Gc/the molar number of all glycan structures in the glycosylation-modified erythropoietin.

In the present application, the term "protein" generally refers to a polymer that is not limited to an amino acid residue of the minimum length. Polypeptides, peptides, oligopeptides, dimers, multimers and analogues are included within this definition. Both intact proteins and their fragments are also included within this definition. This term also comprises post-expression modification forms of proteins, including but not limited to glycosylation, acetylation, phosphorylation, etc. For example, in the present application, a protein may refer to a glycosylation-modified erythropoietin and a fragment thereof.

In the present application, the "CHO-S cells" generally refer to CHO-S Chinese hamster ovarian cells into which for example nucleic acids encoding heterologous polypeptides may be introduced, for example, by transfection. The CHO-S cells include the functional or bioactive variant offsprings that are screened out of the original transfected cells and have the same functional or biological activity.

In the present application, the term "erythropoietin" and its abbreviation "EPO" generally refer to any erythropoietin polypeptide, including but not limited to, recombinant erythropoietin polypeptides, synthesized erythropoietin polypeptides, natural EPO polypeptides, and erythropoietin polypeptides extracted from cells and tissues, with the tissues including but not limited to kidney, liver, urine and blood. For example, the erythropoietin may be an erythropoietin having an amino acid sequence of SEQ ID No: 1. The term "erythropoietin" also refers to the variant of a protein of SEQ ID No: 1, with one or more amino acid residues are altered, deleted, or inserted, and the variant has the same biological activity as an unmodified protein, as reported in EP 1 064 951 or US 6,583,272. The biological activity resulting from the binding of the erythropoietin to the EPO receptor may include: increased reticulocytes and erythrocytes produced by bone marrow cells after administration of the erythropoietin to a subject by injection, as compared to individuals from a non-injected group or a control group.

In the present application, the "glycosylation" generally refers to a process in which a protein or polypeptide may be attached to a carbohydrate moiety at one or more amino positions. Generally, a glycosylation-modified protein or polypeptide contain one or more amino acid residues (for example, arginine or asparagine) to link the carbohydrate moiety. For example, the glycosylation may include a N-linked glycoprotein. The N-linked glycoprotein may contain a glycan structure bound to an N-glycosylation site, for example, a glycan structure attached to the N-glycosylation site at an asparagine residue in a protein. The saccharides found on a glycosylation-modified protein (glycoprotein) include the group consisting of: glucose, galactose, mannose, fucose, N-acetylgalactosamine (GaINAc), N-acetylglucosamine (GIcNAc), and/or sialic acid. For example, the glycosylation to the erythropoietin may be an FA4G4L2S4-containing glycan structure attached to the N-glycosylation site of the erythropoietin, an FA4G4L1S4-containing glycan structure attached to the N-glycosylation site of the erythropoietin, an FA4G4S4-containing glycan structure attached to the N-glycosylation site of the erythropoietin, or a Neu5Gc-containing saccharide structure attached to the N-glycosylation site of the erythropoietin.

In the present application, the ratio of the FA4G4L2S4 structure being 15% "or more" generally refers to that the ratio of the glycan structure of FA4G4L2S4 comprised may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98%, or at least 99% in terms of the glycosylation-modified erythropoietin. For example, the ratio of the glycan structure of FA4G4L2S4 comprised may be at least 18.17% in terms of the glycosylation-modified erythropoietin.

In the present application, the ratio of the FA4G4L12S4 structure being 20% "or more" generally refers to that the ratio of the glycan structure of FA4G4L1S4 comprised may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98%, or at least 99% in terms of the glycosylation-modified erythropoietin. For example, the ratio of the glycan structure of FA4G4L1S4 comprised may be at least 21.58% in terms of the glycosylation-modified erythropoietin.

In the present application, the ratio of the FA4G4S4 structure being 10% "or more" generally refers to that the ratio of the glycan structure of FA4G4S4 comprised may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98%, or at least 99% in terms of the glycosylation-modified erythropoietin. For example, the ratio of the glycan structure of FA4G4S4 comprised may be at least 14.02% in terms of the glycosylation-modified erythropoietin.

In the present application, the molar ratio of the Neu5Gc being 0.5% "or less" generally refers to that the molar ratio of the Neu5Gc may be at most 0.5%, at most 0.4%, at most 0.3%, at most 0.2%, at most 0.1%, at most 0.05%, at most 0.02%, at most 0.01%, or 0% in terms of the glycosylation-modified erythropoietin. For example, the glycosylation-modified erythropoietin may comprise the glycan structure that does not comprise Neu5Gc.

In the present application, "prolonging" the half-life of erythropoietin generally refers to the fact that the glycosylation-modified erythropoietin of the present application has increased proteinase resistance, which may result in an increase of the half-life of the glycosylation-modified erythropoietin *in vitro* (for example, during production, purification and storage) or *in vivo* (for example, after administration to a subject), as compared to a non-glycosylation-modified EPO or a glycosylation-modified EPO that has another glycan structure. For example, after the glycosylation-modified erythropoietin of the present application is administered to a subject, an increase in half-life is exhibited, and compared with an unmodified EPO or a glycosylation-modified EPO having another glycan structure, the glycosylation-modified erythropoietin of the present application may increase the half-life by at least about or at least 1%, 2%, 3%, 4%, 5%, 6%, 7%, 8%, 9%, 10%, 20%, 30%, 40%, 50%, 60%, 70%, 75%, 80%, 90%, 91%, 92%, 93%, 94%, 95%, 96%, 97%, 98%, 99%, 100%, 200%, 300%, 400%, 500% or more. For example, compared with the unmodified EPO or the glycosylation-modified EPO having another glycan structure, the glycosylation-modified erythropoietin of the present application may increase the half-life at least about or at least 6 times, 7 times, 8 times, 9 times, 10 times, 20 times, 30 times, 40 times, 50 times, 60 times, 70 times, 80 times, 90 times, 100 times or more.

### DETAILED DESCRIPTION OF THE INVENTION

In one aspect, the present application provides a glycosylation-modified erythropoietin, comprising 5 glycosylation sites as follows: N24, N30, N38, N83 and N88. A glycosylation site may be used to N-link the carbohydrate chain of the glycan structure of the present application. The glycosylation-modified erythropoietin of the present application is expressed by transfecting a nucleic acid molecule encoding an erythropoietin with an amino acid sequence as set forth in SEQ ID No: 1 into a CHO-S cell. The binding of the glycosylation-modified erythropoietin to an EPO receptor is not enhanced, and in some cases, the binding force to the EPO receptor may be reduced due to the carbohydrate chain structure of the present application. However, the glycosylation-modified erythropoietin of the present application can increase the half-life, thereby increasing the biological activity *in vivo* and increasing the hemoglobin content, erythrocyte level, hematocrit value, and reticulocyte level.

In one aspect, the present application provides a glycan structure of a glycosylation-modified erythropoietin. The glycan structure may comprise one or more FA4G4L2S4 glycan structures.

In another aspect, the present application provides a glycan structure of a glycosylation-modified erythropoietin. The glycan structure may comprise one or more FA4G4L2S4 structures, the ratio(s) of which may be 15% or more. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% in terms of the glycosylation-modified erythropoietin. For example, the ratio of the FA4G4L2S4 glycan structure comprised may be at least 18.17% in terms of the glycosylation-modified erythropoietin.

In another aspect, the present application provides a glycan structure of a glycosylation-modified erythropoietin. The glycan structure may comprise one or more FA4G4L1S4 glycan structures.

In another aspect, the present application provides a glycan structure of a glycosylation-modified erythropoietin. The glycan structure may comprise one or more FA4G4L1S4 structures, the ratio(s) of which may be 20% or more. For example, the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% in terms of the glycosylation-modified erythropoietin. For example, the ratio of the FA4G4L1S4 glycan structure comprised may be at least 21.58% in terms of the glycosylation-modified erythropoietin.

In another aspect, the present application provides a glycan structure of a glycosylation-modified erythropoietin. The glycan structure may comprise one or more FA4G4S4 glycan structures.

In another aspect, the present application provides a glycan structure of a glycosylation-modified erythropoietin. The glycan structure may comprise one or more FA4G4S4 structures, the ratio(s) of which may be 10% or more. For example, the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% in terms of the glycosylation-modified erythropoietin. For example, the ratio of the FA4G4S4 glycan structure comprised may be at least 14.02% in terms of the glycosylation-modified erythropoietin.

In another aspect, the present application provides a glycan structure of a glycosylation-modified erythropoietin. The glycan structure may comprise Neu5Gc, the molar ratio of which may be 0.5% or less.

In another aspect, the present application provides a glycan structure of a glycosylation-modified erythropoietin. The glycan structure may comprise Neu5Gc, the molar ratio of which may be 0.5% or less. For example, the molar ratio of the Neu5Gc may be at most 0.5%, at most 0.4%, at most 0.3%, at most 0.2%, at most 0.1%, at most 0.05%, at most 0.02%, at most 0.01%, or 0% in terms of the glycosylation-modified erythropoietin. For example, the glycosylation-modified erythropoietin may comprise the glycan structure that does no comprise Neu5Gc.

In one aspect, the present application provides a glycan structure of a glycosylation-modified erythropoietin. The glycan structure may comprise one or more FA4G4L2S4 glycan structures and one or more FA4G4L1S4 glycan structures.

In one aspect, the present application provides a glycan structure of a glycosylation-modified erythropoietin. The glycan structure may comprise one or more FA4G4L2S4 structures and one or more FA4G4L1S4 structures. The ratio of the FA4G4L2S4 structure may be 15% or more and the ratio of the FA4G4L1S4 may be 20% or more. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; and the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%, in terms of the glycosylation-modified erythropoietin. For example, the ratio of the FA4G4L2S4 glycan structure comprised may be at least 18.17%; and the ratio of the FA4G4L1S4 glycan structure comprised may be at least 21.58%, in terms of the glycosylation-modified erythropoietin.

In one aspect, the present application provides a glycan structure of a glycosylation-modified erythropoietin. The glycan structure may comprise one or more FA4G4L2S4 glycan structures and one or more FA4G4S4 glycan structures.

In one aspect, the present application provides a glycan structure of a glycosylation-modified erythropoietin. The glycan structure may comprise one or more FA4G4L2S4 structures and one or more FA4G4S4 structures. The ratio of the FA4G4L2S4 structure may be 15% or more, and the ratio of the FA4G4S4 may be 10% or more. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; and the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%, in terms of the glycosylation-modified erythropoietin. For example, the ratio of the FA4G4L2S4 glycan structure comprised may be at least 18.17%; and the ratio of the FA4G4S4 glycan structure comprised may be at least 14.02%, in terms of the glycosylation-modified erythropoietin.

In one aspect, the present application provides a glycan structure of a glycosylation-modified erythropoietin. The glycan structure may comprise one or more FA4G4L1S4 glycan structures and one or more FA4G4S4 glycan structures.

In one aspect, the present application provides a glycan structure of a glycosylation-modified erythropoietin. The glycan structure may comprise one or more FA4G4L1S4 structures and one or more FA4G4S4 structures. The ratio of the FA4G4L1S4 structure may be 20% or more, and the ratio of the FA4G4S4 may be 10% or more. For example, the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%; and the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%, in terms of the glycosylation-modified erythropoietin. For example, the ratio of the FA4G4L1S4 glycan structure comprised may be at least 21.58%; and the ratio of the FA4G4S4 glycan structure comprised may be at least 14.02%, in terms of the glycosylation-modified erythropoietin.

In one aspect, the present application provides a glycan structure of a glycosylation-modified erythropoietin. The glycan structure may comprise one or more FA4G4L2S4 glycan structures and one or more Neu5Gcs.

In one aspect, the present application provides a glycan structure of a glycosylation-modified erythropoietin. The glycan structure may comprise one or more FA4G4L2S4 structures and one or more Neu5Gcs. The ratio of the FA4G4L2S4 may be 15% or more, and the molar ratio of the Neu5Gc may be 0.5% or less. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; and the molar ratio of the Neu5Gc may be at most 0.5%, at most 0.4%, at most 0.3%, at most 0.2%, at most 0.1%, at most 0.05%, at most 0.02%, at most 0.01% or 0%, in terms of the glycosylation-modified erythropoietin. For example, the ratio of the FA4G4L2S4 glycan structure comprised may be at least 18.17%; and the glycan structure may not comprise Neu5Gc, in terms of the glycosylation-modified erythropoietin.

In one aspect, the present application provides a glycan structure of a glycosylation-modified erythropoietin. The glycan structure may comprise one or more FA4G4L1S4 glycan structures and one or more Neu5Gc glycan structures.

In one aspect, the present application provides a glycan structure of a glycosylation-modified erythropoietin. The glycan structure may comprise one or more FA4G4L1S4 structures and one or more Neu5Gcs. The ratio of the FA4G4L1S4 may be 20% or more, and the molar ratio of the Neu5Gc may be 0.5% or less. For example, the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%; and the molar ratio of the Neu5Gc may be at most 0.5%, at most 0.4%, at most 0.3%, at most 0.2%, at most 0.1%, at most 0.05%, at most 0.02%, at most 0.01% or 0%, in terms of the glycosylation-modified erythropoietin. For example, the ratio of the FA4G4L1S4 glycan structure comprised may be at least 21.58%; and the glycan structure may not comprise Neu5Gc, in terms of the glycosylation-modified erythropoietin.

In one aspect, the present application provides a glycan structure of a glycosylation-modified erythropoietin. The glycan structure may comprise one or more FA4G4L2S4 glycan structures, one or more FA4G4L1S4 glycan structures, and one or more FA4G4S4 glycan structures.

In one aspect, the present application provides a glycan structure of a glycosylation-modified erythropoietin. The glycan structure may comprise one or more FA4G4L2S4 structures, one or more FA4G4L1S4 structures, and one or more FA4G4S4 structures. The ratio of the FA4G4L2S4 structure may be 15% or more, the ratio of the FA4G4L1S4 may be 20% or more, and the ratio of the FA4G4S4 may be 10% or more. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%; and the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%, in terms of the glycosylation-modified erythropoietin. For example, the ratio of the FA4G4L2S4 glycan structure comprised may be at least 18.17%; the ratio of the FA4G4L1S4 glycan structure comprised may be at least 21.58%; and the ratio of the FA4G4S4 glycan structure comprised may be at least 14.02%, in terms of the glycosylation-modified erythropoietin.

In one aspect, the present application provides a glycan structure of a glycosylation-modified erythropoietin. The glycan structure may comprise one or more FA4G4L2S4 glycan structures, one or more FA4G4L1S4 glycan structures, and one or more Neu5Gcs.

In one aspect, the present application provides a glycan structure of a glycosylation-modified erythropoietin. The glycan structure may comprise one or more FA4G4L2S4 structures, one or more FA4G4L1S4 structures, and one or more Neu5Gcs. The ratio of the FA4G4L2S4 structure may be 15% or more, the ratio of the FA4G4L1S4 may be 20% or more, and the molar ratio of the Neu5Gc may be 0.5% or less. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%; and the molar ratio of the Neu5Gc may be at most 0.5%, at most 0.4%, at most 0.3%, at most 0.2%, at most 0.1%, at most 0.05%, at most 0.02%, at most 0.01% or 0%, in terms of the glycosylation-modified erythropoietin. For example, the ratio of the FA4G4L2S4 glycan structure comprised may be at least 18.17%; the ratio of the FA4G4L1S4 glycan structure comprised may be at least 21.58%; and the glycan structure may not comprise Neu5Gc, in terms of the glycosylation-modified erythropoietin.

In one aspect, the present application provides a glycan structure of a glycosylation-modified erythropoietin. The glycan structure may comprise one or more FA4G4L2S4 glycan structures, one or more FA4G4L1S4 glycan structures, one or more FA4G4S4 glycan structures, and one or more Neu5Gcs.

In one aspect, the present application provides a glycan structure of a glycosylation-modified erythropoietin. The glycan structure may comprise one or more FA4G4L2S4 structures, one or more FA4G4L1S4 structures, one or more FA4G4S4 glycan structures, and one or more Neu5Gcs. The ratio of the FA4G4L2S4 structure may be 15% or more, the ratio of the FA4G4L1S4 may be 20% or more, the ratio of the FA4G4S4 may be 10% or more, and the molar ratio of the Neu5Gc may be 0.5% or less. For example, the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99%; the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99%; and the molar ratio of the Neu5Gc may be at most 0.5%, at most 0.4%, at most 0.3%, at most 0.2%, at most 0.1%, at most 0.05%, at most 0.02%, at most 0.01% or 0%, in terms of the glycosylation-modified erythropoietin. For example, the ratio of the FA4G4L2S4 glycan structure comprised may be at least 18.17%; the ratio of the FA4G4L1S4 glycan structure comprised may be at least 21.58%; the ratio of the FA4G4S4 glycan structure comprised may be at least 14.02%; and the glycan structure may not comprise Neu5Gc, in terms of the glycosylation-modified erythropoietin.

In one aspect, the present application provides a glycan structure of a glycosylation-modified erythropoietin. The glycan structure may directly or indirectly bind to the N-glycosylation site. For example, the glycan structure may directly bind to the N-glycosylation site by covalent interaction. For example, the monosaccharide of the glycan structure may directly bind to the free-NH₂ group of an asparagine residue at the N-glycosylation site by covalent interaction.

In another aspect, the present application provides a glycan structure of a glycosylation-modified erythropoietin. The glycan structure may bind to the following N-glycosylation sites of the glycosylation-modified erythropoietin: N24, N30, N38, N83 and N88.

In another aspect, the present application provides a glycan structure of a glycosylation-modified erythropoietin. The FA4G4L2S4 structure may bind to the following N-glycosylation sites of the glycosylation-modified erythropoietin: N24, N30, N38, N83 and N88.

In another aspect, the present application provides a glycan structure of a glycosylation-modified erythropoietin. The FA4G4L2S4 structure may bind to the following N-glycosylation sites of the glycosylation-modified erythropoietin: N24, N30, N38, N83 and N88, and the ratio of the FA4G4L2S4 structure may be 15% or more. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin.

In another aspect, the present application provides a glycan structure of a glycosylation-modified erythropoietin. The FA4G4L1S4 structure may bind to the following N-glycosylation sites of the glycosylation-modified erythropoietin: N24, N30, N38, N83 and N88.

In another aspect, the present application provides a glycan structure of a glycosylation-modified erythropoietin. The FA4G4L1S4 structure may bind to the following N-glycosylation sites of the glycosylation-modified erythropoietin: N24, N30, N38, N83 and N88, and the ratio of the FA4G4L1S4 structure may be 20% or more. For example, a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4L1S4 glycan structure comprised may be at least 21.58% may bind to the N-glycosylation site of the glycosylation-modified erythropoietin.

In another aspect, the present application provides a glycan structure of a glycosylation-modified erythropoietin. The FA4G4S4 structure may bind to the following N-glycosylation sites of the glycosylation-modified erythropoietin: N24, N30, N38, N83 and N88.

In another aspect, the present application provides a glycan structure of a glycosylation-modified erythropoietin. The FA4G4S4 structure may bind to the following N-glycosylation sites of the glycosylation-modified erythropoietin: N24, N30, N38, N83 and N88, and the ratio of the FA4G4S4 structure may be 10% or more. For example, a glycosylation modification in which the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4S4 glycan structure comprised may be at least 14.02% may bind to the N-glycosylation site of the glycosylation-modified erythropoietin.

In another aspect, the present application provides a glycan structure of a glycosylation-modified erythropoietin. The glycan structure may comprise Neu5Gc, and may bind to the following N-glycosylation sites of the glycosylation-modified erythropoietin: N24, N30, N38, N83 and N88.

In another aspect, the present application provides a glycan structure of a glycosylation-modified erythropoietin. The glycan structure may comprise Neu5Gc, and may bind to the following N-glycosylation sites of the glycosylation-modified erythropoietin: N24, N30, N38, N83 and N88, and the molar ratio of the Neu5Gc may be 0.5% or less. For example, a glycan structure in which the molar ratio of the Neu5Gc may be at most 0.5%, at most 0.4%, at most 0.3%, at most 0.2%, at most 0.1%, at most 0.05%, at most 0.02%, at most 0.01%, or 0% may bind to the N-glycosylation site of the glycosylation-modified erythropoietin. For example, a glycan structure in which the molar ratio of the Neu5Gc may be 0% may bind to the N-glycosylation site of the glycosylation-modified erythropoietin.

In one aspect, the present application provides a glycan structure of a glycosylation-modified erythropoietin. The FA4G4L2S4 structure and the FA4G4L1S4 structure may bind to the following N-glycosylation sites of the glycosylation-modified erythropoietin: N24, N30, N38, N83 and N88.

In one aspect, the present application provides a glycan structure of a glycosylation-modified erythropoietin. The FA4G4L2S4 structure and the FA4G4L1S4 structure may bind to the following N-glycosylation sites of the glycosylation-modified erythropoietin: N24, N30, N38, N83 and N88, the ratio of the FA4G4L2S4 structure may be 15% or more, and the ratio of the FA4G4L1S4 structure may be 20% or more. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; and a glycosylation modification in which the ratio of the FA4G4L1S4 glycan structure comprised may be at least 21.58% may bind to the N-glycosylation site of the glycosylation-modified erythropoietin.

In one aspect, the present application provides a glycan structure of a glycosylation-modified erythropoietin. The FA4G4L2S4 structure and the FA4G4S4 structure may bind to the following N-glycosylation sites of the glycosylation-modified erythropoietin: N24, N30, N38, N83 and N88.

In one aspect, the present application provides a glycan structure of a glycosylation-modified erythropoietin. The FA4G4L2S4 structure and the FA4G4S4 structure may bind to the following N-glycosylation sites of the glycosylation-modified erythropoietin: N24, N30, N38, N83 and N88, the ratio of the FA4G4L2S4 structure may be 15% or more, and the ratio of the FA4G4S4 structure may be 10% or more. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycosylation modification in which the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 9% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; and a glycosylation modification in which the ratio of the FA4G4S4 glycan structure comprised may be at least 14.02% may bind to the N-glycosylation site of the glycosylation-modified erythropoietin.

In one aspect, the present application provides a glycan structure of a glycosylation-modified erythropoietin. The FA4G4L1S4 structure and the FA4G4S4 structure may bind to the following N-glycosylation sites of the glycosylation-modified erythropoietin: N24, N30, N38, N83 and N88.

In one aspect, the present application provides a glycan structure of a glycosylation-modified erythropoietin. The FA4G4L1S4 structure and the FA4G4S4 structure may bind to the following N-glycosylation sites of the glycosylation-modified erythropoietin: N24, N30, N38, N83 and N88, the ratio of the FA4G4L1S4 structure may be 20% or more, and the ratio of the FA4G4S4 structure may be 10% or more. For example, a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycosylation modification in which the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4L1S4 glycan structure comprised may be at least 21.58% may bind to the N-glycosylation site of the glycosylation-modified erythropoietin; and a glycosylation modification in which the ratio of the FA4G4S4 glycan structure comprised may be at least 14.02% may bind to the N-glycosylation site of the glycosylation-modified erythropoietin.

In one aspect, the present application provides a glycan structure of a glycosylation-modified erythropoietin. The FA4G4L2S4 structure may bind to the following N-glycosylation sites of the glycosylation-modified erythropoietin: N24, N30, N38, N83 and N88, and the glycan structure may comprise Neu5Gc.

In one aspect, the present application provides a glycan structure of a glycosylation-modified erythropoietin. The FA4G4L2S4 structure may bind to the following N-glycosylation sites of the glycosylation-modified erythropoietin: N24, N30, N38, N83 and N88, and the glycan structure may comprise Neu5Gc. The ratio of the FA4G4L2S4 structure may be 15% or more; and the molar ratio of the Neu5Gc may be 0.5% or less. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycan structure in which the molar ratio of the Neu5Gc may be at most 0.5%, at most 0.4%at most 0.3%, at most 0.2%, at most 0.1%, at most 0.05%, at most 0.02%, at most 0.01% or 0% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; and a glycan structure in which the molar ratio of the Neu5Gc may be 0% may bind to the N-glycosylation site of the glycosylation-modified erythropoietin.

In one aspect, the present application provides a glycan structure of a glycosylation-modified erythropoietin. The FA4G4L1S4 structure may bind to the following N-glycosylation sites of the glycosylation-modified erythropoietin: N24, N30, N38, N83 and N88, and the glycan structure may comprise Neu5Gc.

In one aspect, the present application provides a glycan structure of a glycosylation-modified erythropoietin. The FA4G4L1S4 structure may bind to the following N-glycosylation sites of the glycosylation-modified erythropoietin: N24, N30, N38, N83 and N88, and the glycan structure may comprise Neu5Gc. The ratio of the FA4G4L1S4 structure may be 20% or more; and the molar ratio of the Neu5Gc may be 0.5% or less. For example, a glycosylation modification in which the ratio of the FA4G4L1S4 structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycan structure in which the molar ratio of the Neu5Gc may be at most 0.5%, at most 0.4%, at most 0.3%, at most 0.2%, at most 0.1%, at most 0.05%, at most 0.02%, at most 0.01% or 0% may bind to the N-glycosylation site of the glycosylation-modified erythropoietin. For example, a glycosylation modification in which the ratio of the FA4G4L1S4 glycan structure comprised may be at least 21.58% may bind to the N-glycosylation site of the glycosylation-modified erythropoietin; and a glycan structure in which the molar ratio of the Neu5Gc may be 0% may bind to the N-glycosylation site of the glycosylation-modified erythropoietin.

In one aspect, the present application provides a glycan structure of a glycosylation-modified erythropoietin. The FA4G4L2S4 structure, the FA4G4L1S4 structure and the FA4G4S4 structure may bind to the following N-glycosylation sites of the glycosylation-modified erythropoietin: N24, N30, N38, N83 and N88.

In one aspect, the present application provides a glycan structure of a glycosylation-modified erythropoietin. The FA4G4L2S4 structure, the FA4G4L1S4 structure and the FA4G4S4 structure may bind to the following N-glycosylation sites of the glycosylation-modified erythropoietin: N24, N30, N38, N83 and N88. The ratio of the FA4G4L2S4 structure may be 15% or more, the ratio of the FA4G4L1S4 may be 20% or more, and the ratio of the FA4G4S4 structure may be 10% or more. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycosylation modification in which the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; a glycosylation modification in which the ratio of the FA4G4L1S4 glycan structure comprised may be at least 21.58% may bind to the N-glycosylation site of the glycosylation-modified erythropoietin; and a glycosylation modification in which the ratio of the FA4G4S4 glycan structure comprised may be at least 14.02% may bind to the N-glycosylation site of the glycosylation-modified erythropoietin.

In one aspect, the present application provides a glycan structure of a glycosylation-modified erythropoietin. The FA4G4L2S4 structure and the FA4G4L1S4 structure may bind to the following N-glycosylation sites of the glycosylation-modified erythropoietin: N24, N30, N38, N83 and N88, and the glycan structure may comprise Neu5Gc.

In one aspect, the present application provides a glycan structure of a glycosylation-modified erythropoietin. The FA4G4L2S4 structure and the FA4G4L1S4 structure may bind to the following N-glycosylation sites of the glycosylation-modified erythropoietin: N24, N30, N38, N83 and N88, and the glycan structure may comprise Neu5Gc. The ratio of the FA4G4L2S4 structure may be 15% or more, the ratio of the FA4G4L1S4 may be 20% or more, and the molar ratio of the Neu5Gc may be 0.5% or less. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycan structure in which the molar ratio of the Neu5Gc may be at most 0.5%, at most 0.4%, at most 0.3%, at most 0.2%, at most 0.1%, at most 0.05%, at most 0.02%, at most 0.01% or 0% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; a glycosylation modification in which the ratio of the FA4G4L1S4 glycan structure comprised may be at least 21.58% may bind to the N-glycosylation site of the glycosylation-modified erythropoietin; and a glycan structure in which the molar ratio of the Neu5Gc may be 0% may bind to the N-glycosylation site of the glycosylation-modified erythropoietin.

In one aspect, the present application provides a glycan structure of a glycosylation-modified erythropoietin. The FA4G4L2S4 structure, the FA4G4L1S4 structure and the FA4G4S4 structure may bind to the following N-glycosylation sites of the glycosylation-modified erythropoietin: N24, N30, N38, N83 and N88, and the glycan structure may comprise Neu5Gc.

In one aspect, the present application provides a glycan structure of a glycosylation-modified erythropoietin. The FA4G4L2S4 structure, the FA4G4L1S4 structure and the FA4G4S4 structure may bind to the following N-glycosylation sites of the glycosylation-modified erythropoietin: N24, N30, N38, N83 and N88, and the glycan structure may comprise Neu5Gc. The ratio of the FA4G4L2S4 structure may be 15% or more, the ratio of the FA4G4L1S4 may be 20% or more, and the ratio of the FA4G4S4 structure may be 10% or more. For example, a glycosylation modification in which the ratio of the FA4G4L2S4 structure may be at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; a glycosylation modification in which the ratio of the FA4G4L1S4 glycan structure may be at least 20%, at least 21%, at least 22%, at least 23%, at least 24%, at least 25%, at least 26%, at least 27%, at least 28%, at least 29%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; a glycosylation modification in which the ratio of the FA4G4S4 glycan structure may be at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 60%, at least 70%, at least 80%, at least 90%, at least 95%, at least 96%, at least 97%, at least 98% or at least 99% may bind to the N-glycosylation site of the glycosylation-modified erythropoietin; and a glycan structure in which the molar ratio of the Neu5Gc may be at most 0.5%, at most 0.4%, at most 0.3%, at most 0.2%, at most 0.1%, at most 0.05%, at most 0.02%, at most 0.01% or 0% may bind to the N-glycosylation site of the glycosylation-modified erythropoietin. For example, a glycosylation modification in which the ratio of the FA4G4L2S4 glycan structure comprised may be at least 18.17% may bind to the N-glycosylation site of the glycosylation-modified erythropoietin; a glycosylation modification in which the ratio of the FA4G4L1S4 glycan structure comprised may be at least 21.58% may bind to the N-glycosylation site of the glycosylation-modified erythropoietin; and a glycosylation modification in which the ratio of the FA4G4S4 glycan structure comprised may be at least 14.02% may bind to the N-glycosylation site of the glycosylation-modified erythropoietin; and a glycan structure in which the molar ratio of the Neu5Gc may be 0% may bind to the N-glycosylation site of the glycosylation-modified erythropoietin.

In one aspect, the present application provides a preparation method for a glycosylation-modified erythropoietin, comprising: under a condition of expressing the glycosylation-modified erythropoietin, culturing CHO-S cells comprising nucleic acid molecules encoding the glycosylation-modified erythropoietin. With a vector suitable for retention in a mammalian host cell, the encoded erythropoietin with an amino acid sequence as set forth in SEQ ID No: 1 is inserted into an expression vector by using standard techniques. The vector generally comprises the following elements suitable for mammalian host cells: promoters and other "upstream" regulatory elements, origins of replication, ribosome binding sites, transcriptional termination sites, polylinker sites, and selective markers. The vector may also comprise elements that also allow proliferation and maintenance in prokaryotic host cells. For example, appropriate cells or cell lines comprise any cell or cell line derived from mammals (including humans), including Chinese hamster ovary cells (CHO-S cells). Nucleic acid molecules comprising sequences encoding the erythropoietin with an amino acid sequence as set forth in SEQ ID No: 1 are introduced into host cells by using standard transformation or transfection techniques. The host cells are cultured, amplified, screened, transformed or transfected by using publicly available methods (Gething et al., Nature 293, 620-625 (1981): Kaufman et al., Mol Cell. Biol. 5, 1750-1759 (1985); U.S. Patent No.4, 419, 446). The host cells containing sequences encoding erythropoietin DNA are cultured under conditions where the analogues can be expressed. The glycosylation-modified erythropoietin is recovered from the cell culture medium, and then purified by using the method substantially the same as that previously described (WO94/09257). The method for purification may isolate the glycosylation-modified erythropoietin of the present application.

In another aspect, the present application provides a pharmaceutical composition, comprising a therapeutically effective amount of the glycosylation-modified erythropoietin and pharmaceutically acceptable adjuvants, such as diluents, carriers, solubilizers, emulsifiers, preservatives and/or auxiliary agents. The pharmaceutical composition is suitable for the regimen of less than three administrations per week. The composition may be in a liquid or lyophilized form, and comprises diluents (Tris, citrate, acetate or phosphate buffers) having different pH values and ionic strengths, solubilizers such as Tween or polysorbate, carriers such as human serum albumin or gelatin, preservatives such as thimerosal, parabens or benzyl alcohol, antioxidants such as ascorbic acid or sodium metabisulfite, and other components such as lysine or glycine. The selection of a specific composition depends on many factors, including the condition treated, the route of administration and the required pharmacokinetic parameters. For a more extensive review of the ingredients applicable to the pharmaceutical composition, a reference may be made to Remington's Pharmaceutical Sciences, Edition 18, edited by A. R. Gennaro, Mack, Easton, PA (1980). For example, the glycosylation-modified erythropoietin of the present application is formulated in a liquid form with an isotonic sodium chloride/sodium citrate buffer containing human albumin and optionally containing the preservative benzyl alcohol. The composition may comprise analogues having 1, 2, 3, 4 or more additional carbohydrate chains. For example, the pharmaceutical composition of the present application may be administered subcutaneously or intravenously. The finally selected route of administration depends on many factors, and those skilled in the art can determine the final route of administration.

In one aspect, the present application provides an application for the treatment of anemia. The glycosylation-modified erythropoietin provided by the present invention has a unique glycosylation form, and its binding to an EPO receptor can induce a change in the conformation of the EPO receptor to activate multiple downstream signaling pathways, thereby bringing about proliferation and differentiation of the erythrocyte system. By administering the glycosylation-modified erythropoietin to a subject in need of treatment to stimulate erythropoiesis, the hemoglobin content, erythrocyte level, hematocrit value, and reticulocyte level are increased, thereby alleviating anemia such as renal anemia, multiple myeloma anemia and/or carcinogenic anemia, for example, the renal anemia caused by the chronic kidney disease and uremia, the multiple myeloma anemia and the cancerous anemia caused by chemotherapy, etc.

In one aspect, the present application provides a glycosylation-modified erythropoietin and/or a pharmaceutical composition for use in the treatment of anemia. The glycosylation-modified erythropoietin is administered to a subject in need of treatment to stimulate erythropoiesis, in order to increase hemoglobin content, erythrocyte level, hematocrit value, and reticulocyte level, thereby alleviating anemia, for example, renal anemia, multiple myeloma anemia and/or carcinogenic anemia.

In one aspect, the present application provides an application of a glycosylation-modified erythropoietin and/or a pharmaceutical composition in the preparation of a medicament for treating anemia. The glycosylation-modified erythropoietin is administered to a subject in need of treatment to stimulate erythropoiesis, in order to increase hemoglobin content, erythrocyte level, hematocrit value, and reticulocyte level, thereby alleviating anemia, for example, renal anemia, multiple myeloma anemia and/or carcinogenic anemia.

In one aspect, the present application provides a method for prolonging the half-life of erythropoietin. After the glycosylation-modified erythropoietin of the present application is administered to a subject in need of treatment, the elimination rate for the glycosylation-modified erythropoietin in the subject is significantly reduced, and the in vivo half-life of the glycosylation-modified erythropoietin is prolonged.

Not wishing to be bound by any particular theory, the following examples are merely to illustrate the fusion protein, preparation methods and applications and the like according to the present application, and are not intended to limit the scope of the present invention.

### Examples

### Example 1: Vector construction, transfection, cell culture, and purification of glycosylation-modified erythropoietin

The glycosylation-modified erythropoietin of the present application was a glycoprotein containing 165 amino acids. It contained five N-glycosylation sites (N24, N30, N38, N83, and N88). The amino acid sequence of the glycosylation-modified erythropoietin (as set forth in SEQ ID NO:1) was:

APPRLICDSRVLERYLLEAKEAENITTGCNETCSLNENITVPDTKVNFYAWKRMEVGQQAVE

VWQGLALLSEAVLRGQALLVNSSQVNETLQLHVDKAVSGLRSLTTLLRALGAQKEAISPPDAASAAPLRTITA

DTFRKLFRVYSNFLRGKLKLYTGEACRTGD.

Expression vector plasmids pJY08301 were constructed on the skeleton of pIRES plasmids (ClonTech, Cat # 631605, Lot# 8061805A). IL-2 signal peptides (SEQ ID No: 2) and JL14001 genes (SEQ ID No: 3) were synthesized de novo and inserted into MCS1 regions in the pIRES plasmids. Rat glutamine synthetase genes (rat GS) (SEQ ID NO: 4) were partially separated from pGSRK-1 (ATCC, Cat # 63067, Lot# 158451). The rat GS genes were synthesized de novo by integration into 5' fragments and inserted into MCS2 regions in the pIRES plasmids (as shown in FIG. 1).

Parental CHO-S cells were transfected with pJY08301 by using a FuGene 6 transfection reagent. The transfected CHO-S cells (transfectants) were placed in a culture environment with methionine sulfoximine (MSX) at a continuously high concentration, and pressurized and screened with the MSX to obtain a high yield of clones.

By culture and purification, the glycosylation-modified erythropoietin of the present application was obtained. The transfected CHO-S cells were cultured in a CD CHO culture medium to grow to a cell density of 3^{∗}10⁶/mL, and then a production stage was entered. The incubation temperature in the production stage was constant at 37°C, the pH was maintained at 7.2, and the dissolved oxygen was 50%. The produced glycosylation-modified erythropoietin was isolated and purified by ion-exchange purification columns to obtain lot#20190302-2, lot#20190303-2 and lot#20190304-1 as three lots of purified products of the glycosylation-modified erythropoietin of the present application.

The molecules of the glycosylation-modified erythropoietin of the present application were detected by Western-Blot. 100 ng of the purified sample was pipetted and mixed well with a loading buffer, with Darbepoetin (Darbe, lot# 1078765A) as a control. The resulting mixture was heated at 70°C for 10 min, equilibrated to room temperature, and then centrifuged at 4000 rpm for 1 min to collect the supernatant. The sample was added to a 4-12% Bis-Tris precast gel; 1×MOPS SDS running buffer was added; and the supply voltage and time parameters were set to 60 V for 20 min and 180 V for 50 min. After the electrophoresis, the gel was taken out and put into a stem cell transfection kit holding a PVDF membrane, and the parameters of a stem cell transfection instrument were set to 20V for 1 min, 23V for 4 min, and 25V for 2 min for membrane transfer. After the membrane transfer, the PVDF membrane was blocked by use 10% skim milk powder solution; then, an anti-erythropoietin working solution at a ratio of 1:1000 was added; the resulting mixture was incubated at room temperature for 1 h; the PVDF membrane was washed with PBST; then, a goat anti-rabbit IgG working solution at 1:2000 was added; the resulting mixture was incubated at room temperature for 1 h; the PVDF membrane was washed with PBST; and finally, the color was developed and photos were taken.

The results were shown in FIG. 2, the glycosylation-modified erythropoietin of the present application was different from Darbepoetin in molecular weight, and its molecular weight was higher than that of Darbepoetin.

Based on peptide map analysis, the coverage in the peptide map of the glycosylation-modified erythropoietin of the present application was analyzed. 180 µl of the purified sample was pipetted and mixed well with 20 µl of 10x glycoprotein denaturing buffer, with Darbepoetin (Darbe) as a control. The resulting mixture was heated at 100°C for 10 min; then, 20 µl of 10× GlycoBuffer, 40 µl of 10% NP-40, 1 µl of PNGase F, and 119 µl of water were added; and the mixture was incubated at 37°C for 2 h. The sample was then mixed well with 40 µl of guanidine hydrochloride and 8 µl of DTT, and then allowed to stand at 56°C for 40 min. iodoacetamide was added, and the mixture was left at room temperature in dark place for 50 min, and then was replaced with ammonium bicarbonate. Eendonucleases Trpsin, LysC, and GluC were added to the mixture at the ratio of 1:50 (w/w), and the mixture was allowed to stand at 37°C for 21 h. A 15% formic acid solution was added to each sample to terminate the reaction. Mobile phase A: 0.1% formic acid in water; Mobile phase B: 0.1% formic acid in acetonitrile solution. The column temperature was 60°C; the detection wavelength was 214 nm; the sample loading volume was 10 µl; the effective separation time was 2-60 min; and the effective gradient was 5%-50% for the mobile phase B. The mass spectrometry was conducted under the following conditions: positive ion acquisition mode; MSE acquisition; ESI ion source; sample cone voltage: 40 V; capillary voltage: 3 kV; scanning range: 100-2000 m/z; low energy: 6 eV; high energy: 20-50 eV; scanning time: 0.5 sec. The results showed that the coverages in the peptide maps of the glycosylation-modified erythropoietin of the present application and Darbepoetin (Darbe) were up to 85% or more.

The isoelectric point of the glycosylation-modified erythropoietin of the present application was tested by isoelectric focusing (IEF) electrophoresis assay. An IEF precast gel was put in an amphoteric electrolyte buffer at pH 2-6 and allowed to stand overnight. Then, the IEF gel containing the amphoteric electrolyte was placed on an electrophoretic plate precooled to 10°C, and prefocused for 20 min at 700 V to form a pH gradient field. 4.5 µg of purified sample was added to each well, with Darbepoetin (Darbe, lot#1078765A) as a control, and the procedures were set to 500 V for 20 min, 2000 V for 120 min, and 2500 V for 10 min. After electrophoresis, the IEF gel was put in a fixative solution (6 g of TCA, 2.04 g of 5-sulfosalicylic acid, and water added to 50 ml) to stand for 1 h. The IEF gel was rinsed thoroughly with water, and then put in a staining fluid overnight; the aqueous solution was decolorized; and pictures were taken for analysis.

As shown in FIG. 3, Lanes 1-4 were used for Darbepoetin and the three lots of purified products lot#20190302-2, lot#20190303-2, and lot#20190304-1 of the glycosylation-modified erythropoietin of the present application, respectively. The results showed that the number of bands for the purified products of the present application was the same as the number of bands for Darbepoetin, but the content of low isoelectric points (pl) in the bands for the purified products of the present application was high.

The ratio of acidic isomer of the glycosylation-modified erythropoietin in the present application was tested by capillary zone electrophoresis (CZE). With a 3 KDa ultrafiltration concentration tube, the purified sample was exchanged and concentrated into water at a concentration of 1.0 mg/ml, with Darbepoetin (Darbe, lot#1078765A) as a control. Preparation of CZE buffer: 7M Urea, 0.01 M NaCl, 0.01 M sodium acetate, 0.01 M tricine, and 2.5 mM 1,4-Diaminobutane. A PA800 plus instrument was turned on, and a capillary cartridge was installed with each capillary having an inner diameter of 50 microns and with a total length of 110 cm. The procedures were set up as follows: under 20.0 psi, washing with water for 15 minutes; under 20.0 psi, washing with 0.1 M NaOH for 5 minutes; under 20.0 psi, infiltrating in the CZE buffer for 15 min; under 0.7 psi, vacuum injection for 30 se; and at 20.0 KV, separating the sample for 240 min. Sample vial and waste vials were arranged; the cartridge temperature was set to 10°C; the capillary operating temperature was set to 35°C; the detection wavelength was set to 214 nm; and the testing time was set to 214 min.

FIG. 4 showed the Darbepoetin and the three lots of purified products lot#20190302-2, lot#20190303-2 and lot#20190304-1 of the glycosylation-modified erythropoietin of the present application from top to bottom, respectively. The results showed that, compared with Darbepoetin, the purified products of the present invention displayed a higher ratio on the right side of the main peak, indicating a higher ratio of the acid isomers in the purified products of the present application than that of Darbepoetin.

### Example 2: Glycosylated structure analysis of glycosylated-modified erythropoietin

The sialic acid content of the glycosylation-modified erythropoietin of the present application was analyzed by high-performance liquid chromatography (HPLC).

25 µl of each of the three lots of purified products lot#20190302-2, lot#20190303-2 and lot#20190304-1 of the glycosylation-modified erythropoietin of the present application from Example 1 was pipetted, and mixed with 50 µl of 200 mM HCl and 25 µl of water, with Darbepoetin (Darbe, lot# 1078765A) as a control. The resulting mixtures were each heated at 80°C for 2 hours. Neu5Ac (Sigma, 19023-10MG) and Neu5Gc (USP, 1294284) standards were processed respectively according to the method described above. For the final formulations, the concentrations at individual points that make up the Neu5Ac standard curve were 10 µM, 25 µM, 50 µM, 75 µM, and 100 µM, and the concentrations at individual points that make up the Neu5Gc standard curve were 0.2 µM, 0.4 µM, 1 µM, 10 µM, 25 µM, and 50 µM. An aqueous solution containing only HCl and without standards was used as a negative control. Preparation of fluorescent labelling liquid: 0.87 mg of DMB, 44.3 µl of HAc, 29.1 µl of 2-Mercaptoethanol, and 39.6 µl of Na₂S₂O₄ were added to 162.3 µl of water. The sample and the fluorescent labelling liquid were mixed in equal volume, and heated at 50°C for 3 h, and then water was added to terminate the reaction. Mobile phase: 70 ml of methanol and 90 ml of acetonitrile were added to 840 ml of water and mixed well. An HPLC instrument was set as follows: flow rate: 0.5 ml/min, time: 60 min, sample loading volume: 10 µl, excitation light: 373 nm, and emission light: 448 nm. Chromatographic results were acquired to draw corresponding standard curves, and the NeuSAc and Neu5Gc contents in the purified products of the glycosylation-modified erythropoietin of the present applications were calculated, as shown in Table 1 below.

**Table 1 NeuSAc and Neu5Gc contents in samples**

| Sample | Neu5Ac content (mol/mol) | Neu5Gc content (mol/mol) |
|---|---|---|
| Darbe | 21.293 | 0.069 |
| lot#20190302-2 | 21.214 | 0.420 |
| lot#20190303-2 | 20.530 | 0.332 |
| lot#20190304-1 | 20.947 | 0.343 |

The N-linked glycosylation composition of the glycosylation-modified erythropoietin of the present application was analyzed by combined high performance liquid chromatography-mass spectrometry (HPLC-MS). With Zeba centrifuging columns, 15 µg of each of the three lots of purified products lot#20190302-2, lot#20190303-2 and lot#20190304-1 of the glycosylation-modified erythropoietin of the present application from Example 1 was exchanged to water, and dried by spinning evaporation ed; and then HPLC water was added for reconstitution to 2 mg/ml. Darbepoetin (Darbe, lot#1078765A) was taken as a control. 6 µl of 5% RapiGest SF solution and 15.3 µl of water were added to the sample, mixed well, heated at 95°C for 5 min, and then allowed to stand to room temperature. 1.2 µl of Rapid PNGase F was added to the mixture, which was then heated at 50°C for 15 min, and allowed to stand at room temperature for cooling. 12 µl of RapiFlour-MS solution was added to the above glycosyl-released mixture, mixed well, and labeled at room temperature for 10 min, and then 358 µl of acetonitrile was added. A saccharide mixture was extracted with GlycoWorks HILIC µElution columellae, and finally dissolved in 90 µl of SPE elution buffer; and 100 µl of dimethylformamide (DMF) and 210 µl of acetonitrile dilution eluent were added. Mobile phase A: 50 mM ammonium formate, pH 4.4; mobile phase B: acetonitrile. Chromatographic conditions: sample loading volume: 10 µl; excitation light: 265 nm, and emitted light: 425 nm; column temperature: 60°C; effective separation time: 35 min; and elution gradient: 25%-46% for mobile phase A. The mass spectrometer was set up as follows: positive ion resolution mode; MS acquisition; cone voltage: 50 V; capillary voltage: 3.0 kV; desolvation temperature: 250°C; scanning range: 400-3000 m/z; and scanning time: 0.5 sec.

The results were shown in FIG. 5. Compared with Darbepoetin, the glycosylation-modified erythropoietin of the present application displayed a large proportion of 4-branch saccharide types and a larger proportion of N-acetyllactosamine. The ratios of the glycan structures of FA4G4S4, FA4G4L1S4 and FA4G4L2S4 in Darbepoetin were 15.85%, 5.36% and 1.43%, respectively, and the ratios of the glycan structures of FA4G4S4, FA4G4L1S4 and FA4G4L2S4 in the purified samples of the glycosylation-modified erythropoietin of the present application were 14.02%, 21.58% and 18.17%, respectively.

### Example 3: Affinity analysis of glycosylation-modified erythropoietin

The affinity of the glycosylation-modified erythropoietin of the present application to the EPO receptor was tested by a high-throughput molecular interaction analysis platform (GE, Biacore 8K). The temperature of a flow cell was set to 25°C, the temperature of a sample chamber was set to 25°C, and the sensor chip was selected as CM5. Three lots of purified products lot#20190302-2, lot#20190303-2 and lot#20190304-1 of the glycosylation-modified erythropoietin of the present application from Example 1 and EPO receptors (Sinobiological, 10707-H08H) were dispensed into a microdisk, with Darbepoetin (Darbe, lot#1078765A) as a control. The instrument was operated for testing. The affinity assay with the EPO receptor was shown in Table 2 below, and the receptor affinity of the purified samples of the glycosylation-modified erythropoietin of the present application was lower than that of Darbepoetin.

**Table 2 Affinity of samples to EPO receptors**

| Sample | K_{D} (nM) |
|---|---|
| Darbepoetin (lot# 1078765A) | 8.35 |
| lot#20190302-2 | 28.9 |
| lot#20190303-2 | 22.6 |
| lot#20190304-1 | 29.5 |

### Example 4: Cell-based activity analysis of glycosylation-modified erythropoietin

Human blood leukemia cells TF-1(ATCC^{®} CRL-2003^{™}) were recovered and passaged two times. Three lots of purified products lot#20190302-2, lot#20190303-2 and lot#20190304-1 of the glycosylation-modified erythropoietin of the present application from Example 1 were gradiently diluted, Darbepoetin (Darbe, lot#1078765A) was taken as a control, and the dilution range was from 5000 ng/ml to 0.03 ng/ml. 50 µl of each diluted sample was added to a 96-well plate, which was then placed in an incubator with 5% COz at 37°C. The recovered TF-1 cells were transferred to a centrifuge tube, centrifuged at 800 rpm and collected, and then resuspended using a basal culture medium (RPMI-1640 plus 10% FBS); the prepared cell suspension was added to the 96-well plate containing the sample at 1×10⁴ cells per well; after homogeneous mixing, the 96-well plate was placed in an incubator with 5% COz at 37°C for 3 days; MTS (Promega, G3580) was added for color development; and finally the absorbance at each well was detected at 490 nm, and the results were analyzed.

The results were as shown in FIG. 6, and the TF-1 cell proliferation results were shown in Table 3, where the purified samples of the glycosylation-modified erythropoietin of the present application showed an *in vitro* activity increasing with the increase of concentration in the range of 0.52 ng/mL-800 ng/mL, demonstrating a significant dose-effect relationship, namely, the relative titer of the sample = EC₅₀ value of Darbepoetin/EC₅₀ value of the sample. Further, the EC₅₀ value of each purified sample of the glycosylation-modified erythropoietin of the present application was higher than that of Darbepoetin, indicating that the samples of the present application had a lower affinity with the EPO receptor than Darbepoetin.

**Table 3 Effect of samples on TF-1 cell proliferation (N/A indicating no data)**

| Sample | EC₅₀ (ng/ml) | Goodness-of-fit R² | Maximum slope of curve | Minim um | Maxim um | Relative activity |
|---|---|---|---|---|---|---|
| Darbepoetin (lot#1078765A) | 11.37 | 0.9955 | 0.8932 | 0.1906 | 0.9809 | N/A |
| lot#20190302-2 | 21.86 | 0.9961 | 0.8798 | 0.1753 | 0.9036 | 52% |
| lot#20190303-2 | 22.57 | 0.9970 | 0.8631 | 0.1761 | 0.9217 | 51% |
| lot#20190304-1 | 27.97 | 0.9943 | 0.9461 | 0.1903 | 0.9537 | 41% |

### Example 5: In vivo efficacy test of glycosylation-modified erythropoietin

Female immunodeficient mice (CD-1 mice, weighing about 25 g) of 9 weeks old were prepared and administered subcutaneously at a single dose, and three lots of purified products lot#20190302-2, lot#20190303-2 and lot#20190304-1 of the glycosylation-modified erythropoietin of the present application from Example 1 were administered to the mice, with PBS as a blank group (vehicle group), Darbepoetin (Darbe, lot#1078765A) as a control group A, and EPO (Espo, lot#17Y03B) as a control group B, at a dose of 15 µg/kg. Seven days after administration, blood was collected from the heart, and EDTA-Naz was added to blood samples, which were then stored at 4°C. A blood analyzer was used to detect the hemoglobin content, erythrocyte level, hematocrit value and reticulocyte level in the mice.

The results in FIG. 7 showed that 7 days after administration, the mice injected with the samples of the present application demonstrated significantly higher hemoglobin content, erythrocyte level, hematocrit value and reticulocyte level than those in the blank group, and the elevation effect achieved by injecting the samples of the present application was stronger than that achieved by injecting Darbepoetin and EPO.

### Example 6: In vivo half-life analysis of glycosylation-modified erythropoietin

Male SD rats (weighing about 300 g) of 10 weeks old were prepared and administered subcutaneously at a single dose, and three lots of purified products lot#20190302-2, lot#20190303-2 and lot#20190304-1 of the glycosylation-modified erythropoietin of the present application from Example 1 were administered to the rats, with Darbepoetin (Darbe, lot#1078765A) as a control group A and EPO (Espo, lot#17Y03B) as a control group B, at a dose of 2 µg/kg. After administration, 0.3 ml of blood was collected from the tail vein at Hours 4, 10, 24, 32, 48, 72 and 96, allowed to stand at room temperature for 2 hours, and centrifuged at 1200 g for 15 min; the supernatant was stored at -80°C; and the sample content in the blood was detected by an EPO Elisa kit.

The results were shown in FIG. 8 and Table 4, where compared with EPO and Darbepoetin, the time-varying plasma concentration curves and results of each sample of the present application showed significantly reduced *in vivo* elimination rate and prolonged *in vivo* half-life. After the subcutaneous injection of the three lots of purified samples of the present application, the half-lives of the rats were 19.91 hours, 19.15 hours and 20.21 hours, respectively, and the elimination half-lives of the samples of the present application were 30% longer than that of Darbepoetin.

**Table 4 In vivo half-life of samples**

| Sample | Half-life (h) | Time to peak plasma concentration (h) | Peak plasma concentration (ng/ml) | Area under medicament-time curve (h × ng/ml) |
|---|---|---|---|---|
| Darbepoetin (lot#1078765A) | 14.73 | 24 | 2.83 | 115.16 |
| lot#20190302-2 | 19.91 | 25.6 | 2.41 | 110.06 |
| lot#20190303-2 | 19.15 | 24 | 2.50 | 111.14 |
| lot#20190304-1 | 20.21 | 24 | 2.28 | 105.96 |
| EPO | 8.41 | 7.2 | 2.43 | 47.37 |

The foregoing detailed description is provided by way of explanation and examples, and is not intended to limit the scope of the appended claims. Various changes of the embodiments listed in the present application until now would be obvious to those of ordinary skills in the art, and should be kept within the scope of the appended claims and equivalents thereof.

## Claims

1. A glycosylation-modified erythropoietin, comprising a glycan structure binding to an N-glycosylation site, said glycan structure comprising FA4G4L2S4.

2. The glycosylation-modified erythropoietin according to claim 1, wherein a ratio of said FA4G4L2S4 is 15% or more.

3. The glycosylation-modified erythropoietin according to any one of claims 1-2, wherein said glycan structure comprises FA4G4L1S4.

4. The glycosylation-modified erythropoietin according to claim 3, wherein a ratio of said FA4G4L1S4 is 20% or more.

5. The glycosylation-modified erythropoietin according to any one of claims 1-4, wherein said glycan structure comprises FA4G4S4.

6. The glycosylation-modified erythropoietin according to claim 5, wherein a ratio of said FA4G4S4 is 10% or more.

7. The glycosylation-modified erythropoietin according to any one of claims 1-6, wherein said glycan structure comprises Neu5Gc, a molar ratio of which is 0.5% or less.

8. The glycosylation-modified erythropoietin according to any one of claims 1-7, comprising an amino acid sequence as set forth in SEQ ID NO. 1.

9. The glycosylation-modified erythropoietin according to any one of claims 1-8, comprising N-glycosylation sites as follows: N24, N30, N38, N83, and N88.

10. A preparation method for the glycosylation-modified erythropoietin according to any one of claims 1-9, comprising the step of: under a condition of expressing the glycosylation-modified erythropoietin according to any one of claims 1-9, culturing CHO-S cells comprising nucleic acid molecules encoding an amino acid sequence as set forth in SEQ ID NO: 1.

11. A pharmaceutical composition, comprising the glycosylation-modified erythropoietin according to any one of claims 1-9, and a pharmaceutically acceptable adjuvant.

12. Use of the glycosylation-modified erythropoietin according to any one of claims 1-9, and/or the pharmaceutical composition according to claim 11 in the preparation of a medicament for treating anemia.

13. The use according to claim 12, wherein said anemia comprises renal anemia, multiple myeloma anemia and/or carcinogenic anemia.

14. A method for prolonging the half-life of erythropoietin, comprising the step of:
administering the glycosylation-modified erythropoietin according to any one of claims 1-9 to a subject in need thereof.
